# EUROPEAN PATENT APPLICATION

(11) **EP 3 272 876 A1**
(43) Date of publication of application: **24.01.2018**
(21) Application number: 16764945.8
(22) Date of filing: 15.03.2016
(51) Int. Cl.: C12Q 1/48, A61K 45/00, A61P 35/00, A61P 43/00, G01N 33/15, G01N 33/50

(54) **METHOD FOR PREDICTING RESPONSIVENESS TO PHOSPHATIDYLSERINE SYNTHASE 1 INHIBITOR**

(30) Priority: 16.03.2015 JP 2015052062
(71) Applicant: Daiichi Sankyo Company, Limited, Tokyo 103-8426 (JP)
(72) Inventor: YOSHIHAMA, Yohei, Tokyo 140-8710 (JP); INOUE, Tatsuya, Tokyo 134-8630 (JP)
(74) Representative: Arends, William Gerrit
(86) International application number: PCT/JP2016/058052
(87) International publication number: WO 2016/148115

(57) **Abstract**

It is intended to develop a therapeutic strategy for specifically targeting cancer cells having the functional suppression of PSS2. The present inventors have found that PSS1 and PSS2 are in a synthetic lethal relationship, and treatment inhibiting PSS1 serves as a promising approach for the treatment of cancer having the functional suppression of PSS2. It has also been revealed that this therapeutic strategy permits efficient treatment based on companion diagnostics because a PSS1 inhibitor can be administered to a subject selected by using the functional suppression of PSS2 as an index.

## Description

### Technical Field

The present invention relates to a method for predicting responsiveness to the treatment of cancer with a phosphatidylserine synthase 1 inhibitor and a method for selecting a subject for the treatment of cancer by using the functional suppression of phosphatidylserine synthase 2 as an index. The present invention also relates to a method for treating cancer having the functional suppression of phosphatidylserine synthase 2, with a phosphatidylserine synthase 1 inhibitor. The present invention also relates to a therapeutic agent for cancer having the functional suppression of phosphatidylserine synthase 2, comprising a phosphatidylserine synthase 1 inhibitor. The present invention further relates to a method for screening for a compound for use in the treatment of cancer having the functional suppression of phosphatidylserine synthase 2, by using the inhibition of phosphatidylserine synthase 1 as an index.

### Background Art

Phosphatidylserine is an acidic phospholipid having negative charge at its polar head under physiological conditions, and accounts for approximately 5 to 15% of cell membrane phospholipids. Phosphatidylserine is known to be produced by two enzymes, phosphatidylserine synthase 1 (PSS1) and phosphatidylserine synthase 2 (PSS2), in mammalian cells (Non Patent Documents 1 and 2).

PSS1 is an enzyme that produces phosphatidylserine through the exchange of L-serine with the choline moiety of phosphatidylcholine, and PSS2 is an enzyme that produces phosphatidylserine through the parallel base exchanging reaction of phosphatidylethanolamine (Non Patent Documents 1 and 2). PSS1 and PSS2 have approximately 28% of their amino acid sequences in common (Non Patent Documents 3 and 4).

It has been reported that no distinct phenotype was found in PTDSS1 (gene encoding PSS1)-knockout mice, whereas a tendency of male subfertility was observed in PTDSS2 (gene encoding PSS2)-knockout mice. Furthermore, PTDSS1/PTDSS2 double knockout mice become embryonically lethal, suggesting that phosphatidylserine is a molecule critical for survival (Non Patent Document 5).

In recent years, synthetic lethal therapy has received attention as a promising therapy for cancer with patient selection. Attempts have also been made to predict molecules, from amongst metabolic enzymes associated with the biosynthesis of phosphatidylserine, having a synthetic lethal relationship with each other by using *in silico* analysis. PSS2 as well as ethanolaminephosphotransferase (CEPT1) and ethanolamine phosphate cytidylyltransferase (PCYT2), which produce phosphatidylethanolamine, have been proposed as candidates of molecules having a synthetic lethal relationship with PSS1 (Patent Document 1 and Non Patent Document 6). However, the synthetic lethal relationship between these molecules has not been specifically examined.

### Citation List

### Patent Document

Patent Document 1: U.S. Patent Application Publication No. 2012/0283956

### Non Patent Document

Non Patent Document 1: Vance, J.E. et al. Biochim. Biophys. Acta 1831, 543-554 (2013)
Non Patent Document 2: Tomohiro, S. et al. Biochem. J. 418, 421-429 (2009)
Non Patent Document 3: Saito, K. et al. Biochemistry 273, 17199-17205 (1998)
Non Patent Document 4: Stone, S. J. et al. Biochem. J. 342, 57-64 (1999)
Non Patent Document 5: Arikketh et al. J. Biol. Chem. 283, 12888-12897 (2008)
Non Patent Document 6: Folger, O. et al. Molecular System Biology 7, Article No. 501 (2011)

### Summary of Invention

### Problems to be solved by the invention

An object of the present invention is to provide a safe and effective treatment of cancer by synthetic lethal therapy.

### Means of solving the problem

The present inventors have identified a combination of genes in a synthetic lethal relationship by predicting combinations of possible genes in a synthetic lethal relationship *in silico,* and actually examining the predicted combinations of genes using cells. Specifically, the present inventors have found that PSS1 and PSS2 are in a synthetic lethal relationship.

As a result, the present inventors have found that treatment inhibiting PSS1 serves as a promising approach to cancer having the functional suppression of PSS2. It is also evident that this approach permits efficient treatment based on companion diagnostics because a patient can be selected by using the functional suppression of PSS2 as an index.

In addition, the present inventors have successfully developed a novel PSS1 inhibitor screening system with the aim of developing a PSS1 inhibitor useful for this approach, thereby reaching the completion of the present invention.

Thus, the present invention relates to synthetic lethal therapy for specifically targeting cancer cells having the functional suppression of PSS2, and companion diagnostics for the synthetic lethal therapy, and more specifically provides the following aspects:
[1] A method for predicting responsiveness to the treatment of cancer with a phosphatidylserine synthase 1 inhibitor, comprising providing a biological sample derived from a subject, detecting the presence or absence of the functional suppression of phosphatidylserine synthase 2 in the biological sample, and thereby determining that the subject for whom the functional suppression of phosphatidylserine synthase 2 has been detected has responsiveness to the treatment of cancer with a phosphatidylserine synthase 1 inhibitor.
[2] A method for selecting a subject for the treatment of cancer with a phosphatidylserine synthase 1 inhibitor, comprising providing a biological sample derived from a subject, detecting the presence or absence of the functional suppression of phosphatidylserine synthase 2 in the biological sample, and selecting the subject for whom the functional suppression of phosphatidylserine synthase 2 has been detected as a subject for the treatment of cancer with a phosphatidylserine synthase 1 inhibitor.
[3] A method for treating cancer having the functional suppression of phosphatidylserine synthase 2, comprising providing a biological sample derived from a subject, detecting the presence or absence of the functional suppression of phosphatidylserine synthase 2 in the biological sample, and administering a phosphatidylserine synthase 1 inhibitor to the subject for whom the functional suppression of phosphatidylserine synthase 2 has been detected.
[4] A method for treating cancer having the functional suppression of phosphatidylserine synthase 2, comprising administering a phosphatidylserine synthase 1 inhibitor to a subject having the functional suppression of phosphatidylserine synthase 2 in a biological sample derived from the subject.
[5] A method for screening for a compound for use in the treatment of cancer having the functional suppression of phosphatidylserine synthase 2, the method comprising the step of selecting the compound by determining whether or not it inhibits phosphatidylserine synthase 1 as an index.
[6] The method according to any one of [1] to [5], wherein the functional suppression of phosphatidylserine synthase 2 is the homozygous deletion or heterozygous deletion of the phosphatidylserine synthase 2 gene.
[7] A therapeutic agent for cancer having the functional suppression of phosphatidylserine synthase 2, comprising a phosphatidylserine synthase 1 inhibitor.
[8] The therapeutic agent for cancer according to [7], wherein the functional suppression of phosphatidylserine synthase 2 is the homozygous deletion or heterozygous deletion of the phosphatidylserine synthase 2 gene.
[9] A method for detecting the presence or absence of the functional suppression of phosphatidylserine synthase 2 in a biological sample derived from a subject in order to select a subject for the treatment of cancer with a phosphatidylserine synthase 1 inhibitor.

### Advantageous Effects of Invention

According to the present invention, responsiveness to the treatment of cancer with a PSS1 inhibitor can be efficiently predicted by using the functional suppression of PSS2 as an index. According to the present invention, a patient having the functional suppression of PSS2 can be selected by detecting the presence or absence of the functional suppression (e.g., inactivating mutation, decreased expression, homozygous deletion, or heterozygous deletion) of PSS2 in a biological sample derived from a subject, and then, the patient can be subjected to the treatment of cancer with a PSS1 inhibitor. Therefore, outcomes from the treatment of cancer patients can be drastically improved.

### Brief Description of Drawings

[Figure 1] Figure 1 is a diagram showing the state (deletion, diploid, or amplification) of the PTDSS2 gene in various cancer tissues.
[Figure 2] Figure 2 is a diagram showing the state (deletion or amplification) of the PTDSS2 gene in various cancer cell lines. The ordinate depicts the mRNA level of PTDSS2. The abscissa depicts the copy number of the PTDSS2 gene.
[Figure 3] The left diagram of Figure 3 is a diagram showing the copy number variations of the PTDSS2 gene and the EGFR gene in various cancer cell lines. The right diagram of Figure 3 is a diagram showing the mRNA level of PTDSS2 in various cancer cell lines.
[Figure 4] Figure 4 is a diagram showing the influence of PTDSS1 knockdown on the cell growth of PTDSS2 heterozygous deletion cells and PTDSS2 wild-type cells.
[Figure 5] Figure 5 is a diagram showing that in PTDSS2 mutated cells, ZR-75-1, the uptake of serine into a lipid fraction is strongly suppressed by PTDSS1 knockdown, as compared with PTDSS2 wild-type cells, HCT116. The left diagram of Figure 5 shows results of measuring the liquid scintillation count of ¹⁴C-serine in the lipid fraction. The right diagram of Figure 5 shows results of separating the lipid fraction by TLC and conducting autoradiography (PS represents phosphatidylserine).
[Figure 6] Figure 6 is a diagram showing the decrease in the mRNA level of PTDSS2 in PTDSS2-KO HCT116 cells (PTDSS2-KO #3 and PTDSS2-KO #16) prepared in order to confirm the effect of PTDSS1 knockdown on PTDSS2 gene-disrupted (PTDSS2-KO) HCT116 cells.
[Figure 7] Figure 7 is a diagram showing the inhibition of growth and the inhibition of serine uptake into lipid by the RNA interference of PTDSS1 in PTDSS2-KO HCT116 cells (PTDSS2-KO #3 and PTDSS2-KO #16).

### Description of Embodiments

In the present invention, phosphatidylserine synthase 1 (PPS1) is a protein encoded by the PTDSS1 gene. PTDSS1 has the nucleotide sequence represented by SEQ ID NO: 1 of the Sequence Listing. The PSS1 protein has the amino acid sequence represented by SEQ ID NO: 2 of the Sequence Listing. PTDSS1 has been registered in NCBI under Gene ID: 9791 and RefSEQ: accession NM_014754.2 (protein: RefSeq NP_055569.1).

In the present invention, phosphatidylserine synthase 2 (PPS2) is a protein encoded by the PTDSS2 gene. PTDSS2 has the nucleotide sequence represented by SEQ ID NO: 3 of the Sequence Listing. The PSS2 protein has the amino acid sequence represented by SEQ ID NO: 4 of the Sequence Listing. PTDSS2 has been registered in NCBI under Gene ID: 81490 and RefSEQ: accession NM_030783.1_ (protein: RefSeq NP_110410.1).

In the present invention, tumor, malignant tumor, cancer, malignant neoplasm, carcinoma, sarcoma, and the like are collectively referred to as "tumor" or "cancer".

In the present invention, the "subject" means a human or a non-human mammal to be tested by a method for predicting responsiveness to a PSS1 inhibitor. The subject means, for example, a human or a non-human mammal affected by a disease on which a PSS1 inhibitor is expected to have therapeutic effects. Preferred examples of the disease on which a PSS1 inhibitor is expected to have therapeutic effects can include cancer. The non-human mammal can be any organism as long as the organism is classified as a mammal. The non-human mammal includes, for example, monkeys, dogs, cats, cattle, and horses. Preferred examples of the "subject" according to the present invention can include humans and non-human mammals suspected of having cancer, and humans and non-human mammals diagnosed with cancer.

In the present invention, the "biological sample" refers to a tissue, a liquid, or a cell isolated from an individual, or a mixture thereof. Examples thereof can include, but are not particularly limited to, biopsied tumor, spinal fluid, pleural fluid, intra-abdominal fluid, lymph, skin sections, blood, urine, feces, sputum, the respiratory organs, the intestinal tract, the genitourinary tract, saliva, milk, the digestive organs, and cells collected therefrom. Preferred examples of the "biological sample" can include a portion of resected tissue obtained during surgery performed for the purpose or treating cancer, a portion of tissue collected by biopsy or the like from a subject suspected of having cancer, and cells derived from blood, pleural fluid, or intra-abdominal fluid.

The biological sample may be protein extracts or nucleic acid extracts prepared from a tissue, a liquid, or a cell isolated from an individual, or a mixture thereof, etc. The preparation of the protein extracts or the nucleic acid extracts can be carried out by use of a protein preparation method or a nucleic acid preparation method known per se in the art.

The biological sample is preferably a biological sample collected before treatment with a PSS1 inhibitor. Use of such a biological sample enables sensitivity for the PSS1 inhibitor to be predicted before carrying out treatment with the PSS1 inhibitor. As a result, whether or not to apply treatment involving the PSS1 inhibitor to the subject can be determined, i.e., the subject to whom treatment involving the PSS1 inhibitor is applied can be selected.

The "functional suppression of phosphatidylserine synthase 2 (PSS2)" according to the present invention includes deletion, decreased expression, inactivation, and the like, of PSS2. The deletion of PSS2 includes homozygous deletion and heterozygous deletion. The decreased expression of PSS2 includes both decreased expression at the transcriptional level and decreased expression at the translational level. The inactivation of PSS2 typically includes an inactivating mutation of PTDSS2.

Examples of approaches for "detecting the functional suppression of PSS2" according to the present invention include, but are not particularly limited to, the following methods. Methods for extracting genomic DNA or RNA from a biological sample derived from a subject are not particularly limited, and an approach known in the art can be appropriately selected for use. Examples of the method for extracting genomic DNA include the SDS phenol method (a method which involves denaturing proteins in tissues preserved in a urea-containing solution or ethanol, with a proteolytic enzyme (proteinase K), a surfactant (SDS), and phenol, and extracting DNA from the tissues by precipitation with ethanol), and DNA extraction methods using Clean Columns (registered trademark, manufactured by NexTtec Biotechnologie GmbH), AquaPure (registered trademark, manufactured by Bio-Rad Laboratories, Inc.), ZR Plant/Seed DNA Kit (manufactured by Zymo Research Corp.), AquaGenomic Solution (registered trademark, manufactured by MoBiTec GmbH), prepGEM (registered trademark, manufactured by ZyGEM NZ Ltd.), Buccal Quick (registered trademark, manufactured by TrimGen Corp.), or the like. Examples of the method for extracting RNA include extraction methods using phenol and chaotropic salt (more specifically, extraction methods using a commercially available kit such as TRIzol (manufactured by Invitrogen Corp.) or ISOGEN (manufactured by Wako Pure Chemical Industries, Ltd.)), and methods using other commercially available kits (RNA Prep Total RNA Extraction Kit (manufactured by Beckman Coulter Inc.), RNeasy Mini (manufactured by Qiagen N.V.), RNA Extraction Kit (manufactured by Pharmacia Biotech), etc.). Examples of reverse transcriptases for use in further preparing cDNA from the extracted RNA include, but are not particularly limited to, reverse transcriptase derived from retrovirus such as RAV (Rous associated virus) or AMV (avian myeloblastosis virus), and reverse transcriptase derived from mouse retrovirus such as MMLV (Moloney murine leukemia virus).

### - Detection of deletion of PSS2 -

The deletion of PSS2 can be confirmed by analyzing the genotype of PTDSS2. The analysis of the genotype of PTDSS2 can employ any method known in the art. For example, the analysis of the genotype can be carried out by detecting the copy number variation (CNV) of PTDSS2 DNA. CNV refers to a phenomenon in which 1 kb or more of genomic DNA on the chromosomes is of 1 copy or less or 3 copies or more, though being of 2 copies in a normal human somatic cell, i.e., the diploid genome. It can be considered that: when the gene copy number is 1 copy or less, the genomic DNA is deleted; and when the gene copy number is 3 copies or more, the genomic DNA is duplicated. The detection of CNV can be carried out by use of a method known in the art. Specific examples of such a method can include an array CGH method, a single base polymorphism (SNP) array method, quantitative real time polymerase chain reaction (qPCR), a multiplex ligation-dependent probe amplification method, next-generation sequencing, and digital PCR.

### - Detection of decreased expression of PSS2 -

In the present specification, the term "decreased expression" means that the expression level is lower when compared with a control (e.g., an average expression level of normal subjects, or an expression level in noncancer tissue of the same patient).

Specifically, the analysis of the expression of the PTDSS2 gene in a biological sample can be carried out, for example, by measuring the amount of mRNA which is a transcript of the gene to be assayed, or measuring the amount of a protein which is a product of the gene to be assayed.

A gene expression detection method known in the art can be used as a method for measuring the amount of mRNA. For example, the measurement of the amount of mRNA can be carried out by use of a number of molecular biological approaches such as Northern blotting, dot blot, polymerase chain reaction (PCR), reverse transcription PCR (RT-PCR), quantitative RT-PCR, hybridization, DNA array method, next-generation sequencing, and digital PCR. Also, the measurement of the amount of mRNA can be carried out by a method known in the art using, as a probe, a polynucleotide having a DNA sequence that hybridizes under stringent conditions to the gene to be assayed. For example, for the preparation of a probe, the probe is appropriately bound to a label such as a fluorescent label. This labeled probe is hybridized to mRNA isolated and purified from a biological sample or cDNA synthesized from the mRNA. Then, fluorescence intensity derived from the hybridized probe can be measured to thereby detect the amount of mRNA of the gene to be assayed. The probe may be immobilized, for use, onto a support such as glass beads or a glass substrate. Specifically, the probe can also be used in the form of a DNA array or a DNA chip in which the probe prepared for the gene to be assayed is immobilized on a support. The support is not particularly limited as long as the support permits immobilization of polynucleotides. The support may have any shape or may be made of any material. General examples of the support can include: inorganic materials such as glass plates, silicon wafers, and resins; nitrocellulose as a natural polymer material; and nylon as a synthetic polymer material.

A commercially available DNA chip or DNA array can be used. The polynucleotide to be immobilized onto a support may be a synthetic oligonucleotide. A nucleic acid derivative, which is capable of being fluorescently labeled, may be introduced into the sequence of the synthetic oligonucleotide. Any of the Affymetrix DNA chip technique, which can synthesize the oligonucleotide of interest on a support, and the Stanford DNA chip technique, which immobilizes a synthesized DNA fragment as a DNA probe by spotting, can be used. Alternatively, the desired polynucleotide may be immobilized by spotting onto the surface of the upper end of columns of the 3D-Gene product (manufactured by Toray Industries, Inc.) which acts as a support having a three-dimensional structure. The phrase "hybridize under stringent conditions" means that the hybridization is maintained even after washing treatment at 42°C with, for example, a buffer solution containing 1 × SSC (0.15 M NaCl and 0.015 M sodium citrate) and 0.1% sodium dodecyl sulfate (SDS). In addition to the temperature conditions described above, various other factors influence the stringency of hybridization. Those skilled in the art can achieve stringency, which is equivalent to the stringency of hybridization shown above as an example, by combining various factors.

A probe and a primer set for quantitatively detecting mRNA or cDNA derived from the PTDSS2 gene are not particularly limited as long as the mRNA or the cDNA can be specifically detected. An oligonucleotide consisting of 12 to 26 bases is preferred. Such a probe and a primer set can be appropriately designed on the basis of information on the nucleotide sequence of the gene to be assayed. Then, the oligonucleotide having the determined sequence can be synthesized according to routine methods using, for example, a DNA synthesizer. Alternatively, the desired primer or probe may be selected, for use, from commercially available primers or probes for gene detection.

A protein assay method known in the art can be used as a method for quantitatively measuring the PSS2 protein. For example, various methods using an antibody against the PSS2 protein can be applied thereto. Specific examples thereof can include Western blot, enzyme-linked immunosorbent assay (ELISA), and radioimmunoassay (RIA).

The antibody against the PSS2 protein is directed to the PSS2 protein as an antigen. A human antibody, a mouse antibody, a rat antibody, a rabbit antibody, a sheep antibody, or the like can be appropriately used as long as the antibody specifically binds to the antigen. The antibody may be a polyclonal antibody or may be a monoclonal antibody. A monoclonal antibody is preferred because homogeneous antibodies can be stably produced. The polyclonal antibody and the monoclonal antibody can be prepared by methods well known to those skilled in the art. Alternatively, the desired antibody may be selected, for use, from commercially available antibodies.

Monoclonal antibody-producing hybridomas can be prepared, generally, by use of a technique known in the art as follows: the antigen of interest or cells expressing the antigen of interest is used as a sensitizing antigen. The desired animal is immunized with this sensitizing antigen according to an ordinary immunization method, and the resulting immunocytes can be fused with known parent cells by an ordinary cell fusion method, followed by the selection of cells (hybridoma cells) producing the desired monoclonal antibody by an ordinary screening method to prepare the hybridomas. The preparation of the hybridomas can be carried out according to, for example, the method of Milstein et al. ("Methods of Enzymology", 1981, Vol. 73, p. 3-46).

In this context, the preparation of the monoclonal antibody can employ the PSS2 protein or a fragment thereof as an antigen. Those skilled in the art can easily obtain the PSS2 protein or the fragment thereof according to a method described in a textbook, for example, Sambrook et al. ed., "Molecular Cloning A Laboratory Manual", 2nd edition, Vol. 1-3, Cold Spring Harbor Laboratory Press, N.Y., 1989.

For the quantification of the PSS2 protein, the protein, the fragment thereof, or the antibody may be immobilized, for use, on a support. The support is not limited as long as the support permits immobilization of proteins. General examples thereof can include: inorganic materials such as glass plates, silicon wafers, and resins; nitrocellulose as a natural polymer material; and nylon and polystyrene as synthetic polymer materials.

The PSS2 protein can also be detected by use of mass spectrometry (MS). Particularly, liquid chromatography-mass spectrometry (LC/MS) is sensitive and therefore advantageous. The measurement by mass spectrometry can be performed, for example, by preparing proteins from a biological sample, labeling the proteins, fractionating the proteins, subjecting the fractionated proteins to mass spectrometry, and identifying the PSS2 protein from the mass spectrometry value. An isotope labeling reagent known in the art can be used as the label. An appropriate labeling reagent can be obtained as a commercially available product. Also, the fractionation can be performed by a method known in the art and can be performed using, for example, a commercially available strongly cationic column.

Meanwhile, it is known in the art that promoter hypermethylation is partly responsible for the decreased expression of a gene. Thus, it is also possible to detect the presence or absence of the functional suppression of PSS2 by using the methylation of the PSS2 gene promoter as an index. The detection of promoter methylation can employ a method known in the art, for example, a method of directly detecting, by nucleotide sequence determination, changes in nucleotide sequence after treatment with bisulfite having the activity of converting methylated cytosine to uracil, or a method of indirectly detecting this change using a restriction endonuclease that can recognize (or cleave) a nucleotide sequence before the bisulfite treatment, but cannot recognize (or cleave) the nucleotide sequence after the bisulfite treatment.

### - Detection of mutation of PSS2 -

In the present invention, the phrase "detect mutation" means detecting mutation of genomic DNA, as a rule. If the mutation of genomic DNA is reflected in a base change in a transcript or an amino acid change in a translation product, the phrase "detect mutation" is also meant to include detecting the change in the transcript or the translation product (i.e., indirect detection).

In a preferred aspect, the method of the present invention is a method for detecting mutation by directly determining the nucleotide sequence of the PTDSS2 gene region in a biological sample derived from a subject. In the present specification, the "PTDSS2 gene region" means a given region of genomic DNA comprising the PTDSS2 gene. The region comprises expression control regions (e.g., a promoter region and an enhancer region) of the PTDSS2 gene, a 3'-terminal noncoding region of the PTDSS2 gene, and the like. Mutation in these regions may influence, for example, the transcriptional activity of the PTDSS2 gene.

The determination of the nucleotide sequence can be performed by a method generally known to those skilled in the art, such as the Maxam-Gilbert method or the Sanger method.

The determined nucleotide sequence of DNA or cDNA can be compared with a control (e.g., the nucleotide sequence of DNA or cDNA derived from a noncancer tissue of the same patient) thereby to determine the presence or absence of the mutation of the PTDSS2 gene region in the test tissue.

The method for detecting mutation of the PTDSS2 gene region can be performed by various methods capable of detecting mutation, in addition to the method which involves directly determining the nucleotide sequence of DNA or cDNA.

For example, the mutation detection according to the present invention can be performed by a method as mentioned below. First, a DNA or cDNA sample is prepared from a biological sample. Subsequently, an oligonucleotide probe having a nucleotide sequence complementary to a nucleotide sequence containing the mutation of the PTDSS2 gene region is prepared by labeling with a reporter fluorescent dye and a quencher fluorescent dye. Then, the oligonucleotide probe is hybridized to the DNA sample. Further, the nucleotide sequence containing the mutation of the PTDSS2 gene region is amplified by using the DNA sample hybridized with the oligonucleotide probe as a template. Then, fluorescence emitted by the reporter fluorescent dye, due to the decomposition of the oligonucleotide probe resulting from amplification, is detected. Subsequently, the detected fluorescence is compared with a control. Examples of such a method include the double dye probe method, i.e. the so-called TaqMan(R) probe method.

In another method, a DNA or cDNA sample is prepared from a biological sample. Subsequently, in a reaction system involving an intercalator that emits fluorescence when inserted between the strands of a DNA duplex, the nucleotide sequence containing the mutation of the PTDSS2 gene region is amplified by using the DNA sample as a template. Then, the temperature of the reaction system is changed, and variation in the intensity of fluorescence emitted by the intercalator is detected. The detected variation in the intensity of fluorescence in conjunction with the change in the temperature is compared with a control. Examples of such a method include HRM (high resolution melting).

In a further alternative method a DNA or cDNA sample is firstly prepared from a biological sample. Subsequently, DNA containing the mutation site of the PTDSS2 gene region is amplified. Further, the amplified DNA is cleaved with restriction enzymes. Subsequently, the DNA fragment is separated according to its size. Subsequently, the size of the detected DNA fragment is compared with a control. Examples of such a method include a method based on restriction fragment length polymorphism (RFLP), and PCR-RFLP.

In a further alternative method, first, a DNA or cDNA sample is prepared from a biological sample. Subsequently, DNA containing the mutation site of the PTDSS2 gene region is amplified. Further, the amplified DNA is dissociated into single-stranded DNA. Subsequently, the single-stranded DNA thus obtained by dissociation is separated on a nondenaturing gel. The mobility of the separated single-stranded DNA on the gel is compared with a control. Examples of such a method include PCR-SSCP (single-strand conformation polymorphism).

In a further alternative method, first, a DNA or cDNA sample is prepared from a biological sample. Subsequently, DNA containing the mutation site of the PTDSS2 gene region is amplified. Further, the amplified DNA is separated on a gel where the concentration of a DNA denaturant is gradually elevated. Subsequently, the mobility of the separated DNA on the gel is compared with a control. Examples of such a method include denaturant gradient gel electrophoresis (DGGE).

A further alternative method employs DNA containing the mutation site of the PTDSS2 gene region prepared from a biological sample, and a substrate on which an oligonucleotide probe that hybridizes to the DNA is immobilized. Examples of such a method include DNA array methods.

In a further alternative method a DNA or cDNA sample is firstly prepared from a biological sample. Also, an "oligonucleotide primer having a nucleotide sequence complementary to one base immediately downstream from the base of the mutation site of the PTDSS2 gene region, and its downstream nucleotide sequence" is prepared. Subsequently, ddNTP primer extension reaction is performed by using the DNA as a template and by using the primer. Subsequently, the primer extension reaction product is applied to a mass spectrometer to conduct mass measurement. Subsequently, the genotype is determined from the mass measurement results. Subsequently, the determined genotype is compared with a control. Examples of such a method include MALDI-TOF/MS.

In a further alternative method a DNA or cDNA sample is firstly prepared from a biological sample. Subsequently, an oligonucleotide probe consisting of 5'-"a nucleotide sequence complementary to the base of the mutation site of the PTDSS2 gene region, and its upstream nucleotide sequence"-"a nucleotide sequence that hybridizes to neither one base immediately downstream from the mutation site of the PTDSS2 gene region nor its downstream nucleotide sequence"-3' (flap) is prepared. Also, an "oligonucleotide probe having a nucleotide sequence complementary to the base of the mutation site of the PTDSS2 gene region and its downstream nucleotide sequence" is prepared. Subsequently, these two types of oligonucleotide probes are hybridized to the prepared DNA. Subsequently, the hybridized DNA is cleaved with a single-stranded DNA cleaving enzyme to liberate the flap. In the present invention, examples of the single-stranded DNA cleaving enzyme include, but are not particularly limited to, cleavase. In this method, subsequently, a reporter fluorescence- and quencher fluorescence-labeled oligonucleotide probe having a sequence complementary to the flap is hybridized to the flap. Subsequently, the intensity of the generated fluorescence is measured. Subsequently, the measured fluorescence intensity is compared with a control. Examples of such a method include the Invader method.

In a further alternative method a DNA or cDNA sample is firstly prepared from a biological sample. Subsequently, DNA containing the mutation site of the PTDSS2 gene region is amplified. Then, the amplified DNA is dissociated into single strands. Only one of the single strands of the dissociated DNA is then isolated. Subsequently, a base extension reaction is performed, base by base, from near the base of the mutation site of the PTDSS2 gene region. Pyrophosphoric acid formed during the reaction brings about enzymatic emission of light, and the intensity of the light is measured. Then, the measured fluorescence intensity is compared with a control. Examples of such a method include pyrosequencing.

In a further alternative method a DNA or cDNA sample is firstly prepared from a biological sample. Subsequently, DNA containing the mutation site of the PTDSS2 gene region is amplified. Subsequently, an "oligonucleotide primer having a nucleotide sequence complementary to one base immediately downstream from the base of the mutation site of the PTDSS2 gene region, and its downstream nucleotide sequence" is prepared. Subsequently, a single base extension reaction is performed by using the amplified DNA as a template and the prepared primer in the presence of fluorescently labeled nucleotides. Then, the degree of polarization of fluorescence is measured. Subsequently, the measured degree of polarization of fluorescence is compared with a control. Examples of such a method include AcycloPrime.

In a further alternative method a DNA or cDNA sample is firstly prepared from a biological sample. Subsequently, DNA containing the mutation site of the PTDSS2 gene region is amplified. Subsequently, an "oligonucleotide primer having a nucleotide sequence complementary to one base immediately downstream from the base of the mutation site of the PTDSS2 gene region, and its downstream nucleotide sequence" is prepared. Subsequently, a single base extension reaction is performed by using the amplified DNA as a template and the prepared primer in the presence of fluorescently labeled nucleotides. Subsequently, the base species used in the single base extension reaction is determined. Subsequently, the determined base species is compared with a control. Examples of such a method include SNuPE. If the mutation results in an amino acid change (e.g., substitution, deletion, or insertion) in the PTDSS2 protein, the sample prepared from the biological sample may be a protein. In such a case, the mutation detection can employ a method using a molecule (e.g., an antibody) specifically binding to a site with the amino acid change caused by the mutation.

In the present invention, it has been found that PSS1 and PSS2 are in a synthetic lethal relationship in cancer cells, and the inhibition of PSS1 in cancer cells having the functional suppression of PSS2 is capable of suppressing the growth of the cancer cells. On the basis of this finding, responsiveness to the treatment of cancer with a compound inhibiting PSS1 can be evaluated by using the functional suppression of PSS2 as an index. Thus, the present invention provides a method for predicting responsiveness to the treatment of cancer with a PSS1 inhibitor, comprising using a biological sample derived from a subject, detecting the presence or absence of the functional suppression of PSS2 in the biological sample, and thereby determining that the subject for which the functional suppression of PSS2 has been detected has responsiveness to the treatment of cancer with a PSS1 inhibitor.

The subject for whom the functional suppression of PSS2 has been detected in this way is also suitable for the treatment of cancer with a PSS1 inhibitor. A patient who is to receive the treatment of cancer with a PSS1 inhibitor, or otherwise can be selected by using the functional suppression of PSS2 as an index, and efficiently treated. Thus, the present invention provides a method for selecting a subject for the treatment of cancer with a PSS1 inhibitor, comprising using a biological sample derived from the subject, detecting the presence or absence of the functional suppression of PSS2 in the biological sample, and selecting the subject for whom the functional suppression of PSS2 has been detected as a subject for the treatment of cancer with a PSS1 inhibitor.

In the present invention, the target cancer is not particularly limited, and every cancer can be targeted. Examples of the cancer confirmed to have the functional suppression of PSS2 include, but are not limited to, testicular germ cell tumor, ovary cancer, bladder cancer, lung cancer, breast cancer, and esophageal cancer. The target cancer may be, for example, colon cancer, prostate cancer, stomach cancer, uterine cervical cancer, endometrial cancer, uterine body cancer, kidney cancer, thyroid gland cancer, squamous cell carcinoma, leukemia, osteosarcoma, melanoma, glioblastoma, neuroblastoma, head and neck cancer, testicular tumor, large intestinal cancer, blood cancer, retinoblastoma, or pancreatic cancer.

Meanwhile, it has been reported that LOH (loss of heterozygosity) of chromosome 11p15.5, where the PTDSS2 gene resides, correlates with the poor prognosis of non-small cell lung cancer (Journal of Clinical Oncology, Vol 20, No 5 (March 1), 2002: pp 1353-1360). Therefore, the present inventors believe that non-small cell lung cancer having LOH of chromosome 11p15.5 may be targeted according to the present invention. Furthermore, PSS2 deletion has been observed to tend to overlap with neither EGFR mutation nor ALK fusion gene mutation in lung adenocarcinoma. Hence, the present invention provides a novel treatment method for a lung adenocarcinoma patient without EGFR mutation or ALK mutation, for which a chemotherapeutic agent is currently the only drug treatment option.

In an alternative aspect, the present invention provides a method for treating cancer having the functional suppression of PSS2, comprising using a biological sample derived from a subject, detecting the presence or absence of the functional suppression of PSS2 in the biological sample, and administering a PSS1 inhibitor to the subject for whom the functional suppression of PSS2 has been detected. Also, the present invention provides a therapeutic agent for cancer having the functional suppression of PSS2, the therapeutic agent comprising a PSS1 inhibitor.

The "PSS1 inhibitor" according to the present invention is not particularly limited and may be a compound known in the art or may be a compound identified by the screening mentioned later.

The PSS1 inhibitor can be orally administered or parenterally administered (e.g., intravenously administered, intraarterially administered, or locally administered) in various forms such as tablets, powders, granules, capsules, and solutions according to its characteristics to a cancer patient. The dose is not particularly limited as long as the dose is an amount effective for treating cancer by inhibiting PSS1. The dose can be appropriately selected according to the properties of the compound as well as the age, body weight, symptoms, and health conditions of the cancer patient, the degree of progression of cancer, etc. The dosing frequency is not particularly limited and can be appropriately selected according to purpose. For example, a daily dose may be administered once a day or may be administered in several divided portions per day. In the case of administering the compound inhibiting PSS1 to a human, the dose range is approximately 0.01 mg/kg body weight to approximately 500 mg/kg body weight, preferably approximately 0.1 mg/kg body weight to approximately 100 mg/kg body weight, per day. For administration to a human the daily dose is, preferably, administered once a day or in two to four divided portions per day, and this administration is preferably repeated at an appropriate interval. Also, the daily dose may exceed the amount described above, if necessary, at a physician's discretion.

### <Method for screening for PSS1 inhibitor for use in the treatment of cancer>

The present invention provides a method for screening for a compound for use in the treatment of cancer having the functional suppression of PSS2, the method comprising the step of selecting the compound by determining whether or not it inhibits PSS1 as an index.

Since PSS1 is an enzyme that produces phosphatidylserine, the screening system of the present invention can be established by using a test to confirm the production of phosphatidylserine by PSS1.

Examples of the test compound to be applied to the screening system of the present invention include, but are not particularly limited to, synthetic low molecular compound libraries, expression products of gene libraries, peptide libraries, siRNA, antibodies, substances released by bacteria, extracts and culture supernatants of cells (microbes, plant cells, and animal cells), purified or partially purified polypeptides, marine organisms, plant- or animal-derived extracts, and random phage peptide display libraries.

The PSS1 inhibitor identified by the screening of the present invention is mixed with a pharmaceutically acceptable carrier, and the mixture can be formulated by a pharmaceutical method known in the art to prepare a pharmaceutical product. Examples of the pharmaceutically acceptable carrier include, but are not limited to, sterile water, saline, plant oils, solvents, bases, emulsifiers, suspending agents, surfactants, stabilizers, flavors, aromatic substances, excipients, vehicles, antiseptics, binders, diluents, tonicity agents, soothing agents, bulking agents, disintegrants, buffers, coating agents, lubricants, colorants, sweeteners, thickeners, corrigents, solubilizers, and other additives.

### Examples

### [Test Example 1] Search for gene family having synthetic lethal relationship

### (In silico prediction)

Approximately 9000 genes found to be homozygously deleted in cancer tissues were extracted from data on gene copy numbers in various cancer tissues derived from cancer patients using The Cancer Genome Atlas (TCGA) Data (provided by National Cancer Institute).

Next, from among the extracted genes, genes that belonged to certain gene families and were found to be homozygously deleted in 1% or more of any cancer selected from pancreatic cancer, lung cancer, breast cancer, kidney cancer, and glioblastoma multiforme were further extracted. From among these genes, genes were further extracted such that two functionally overlapping genes formed a gene family and no literature information had reported that the two genes were not lethal even by homozygous deletion in mice or the like. The extracted gene families were 26 families.

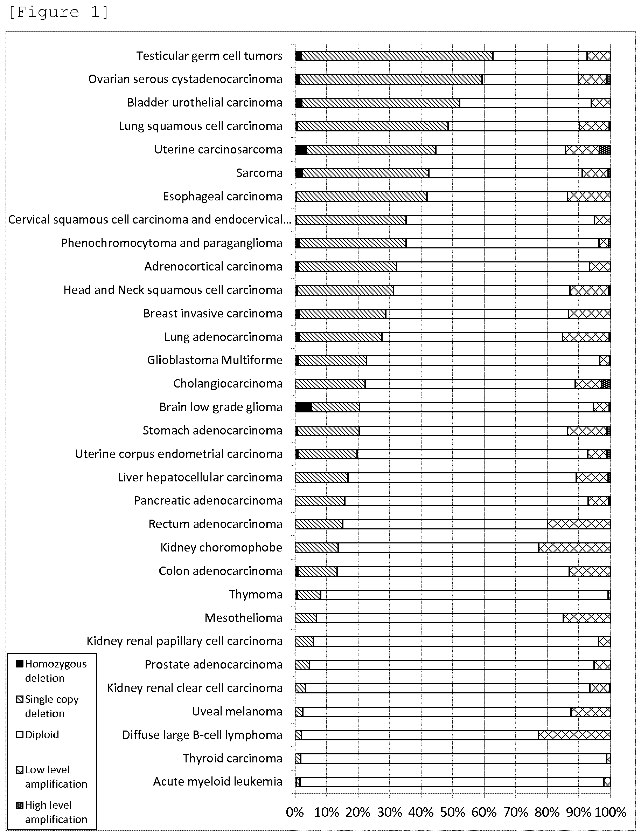

Figure 1 shows the state (homozygous deletion, heterozygous deletion, diploid, or amplification) of PTDSS2, which was a gene belonging to one of the selected 26 families, in various cancers.

### (Search for PTDSS2 gene deletion cell line)

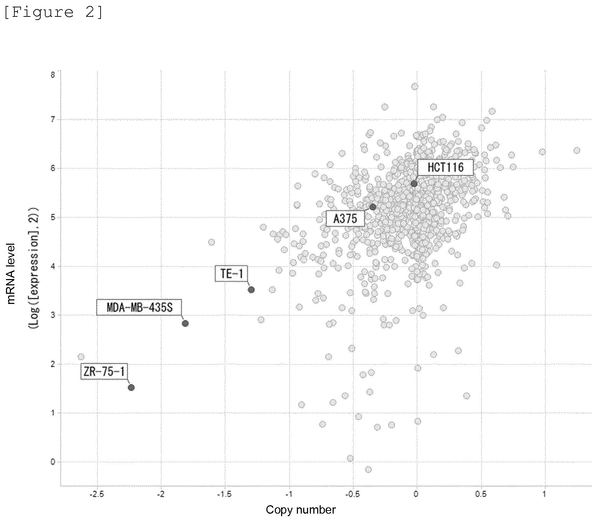

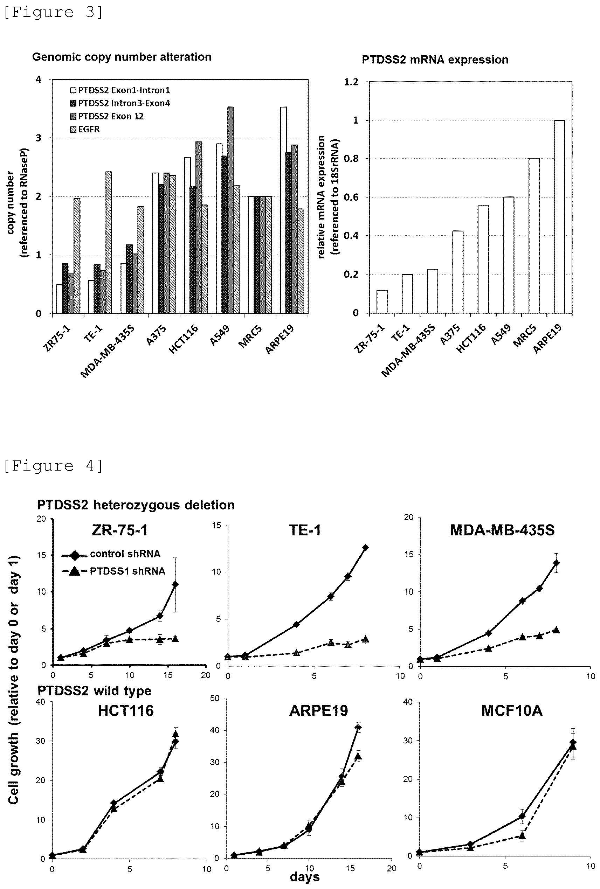

PTDSS2, a gene extracted in the *in silico* prediction, was analyzed for its state in various cancer cell lines using the Cancer Cell Line Encyclopedia (CCLE) database (provided by Broad Institute) (Figure 2). A decrease in the copy number and mRNA level of PTDSS2 was confirmed in ZR-75-1 cells, MDA-MB-435S cells, and TE-1 cells (Figure 3) .

### (Inhibition of growth by PTDSS1 knockdown in PTDSS2 deletion line)

The influence of PTDSS1 knockdown on cell lines (HCT-116 cells, ARPE19 cells, and MCF10A cells) having no deletion in the PTDSS2 gene and PTDSS2 heterozygous deletion cell lines (ZR-75-1 cells, MDA-MB-435S cells, and TE-1 cells) was studied by the RNA interference of PTDSS1.

The cells of each line except for MCF10A were cultured in a medium supplemented with 10% fetal bovine serum (FBS). The combination of the cells and the medium is as described below.
ZR-75-1 cells: RPMI-1640 (Gibco: A10491-01)
MDA-MB-435S cells, TE-1 cells, and ARPE cells: RPMI-1640
(Gibco: 11875-093)
HCT116 cells: McCoy's 5A modified medium

The MCF10A cells were cultured in a MGBM medium supplemented with an additive (Lonza/Clonetics Corporation: Catalog No. CC-3150 (other than GA1000)) and further supplemented with 100 ng/ml cholera toxin.

In order to diminish the PTDSS1 protein expression, a plasmid for short hairpin (sh) RNA expression lentivirus vector preparation (PTDSS1 MISSION shRNA Plasmid DNA (Product number: SHCLND-NM_014754 TRC number: TRCN0000045808)) was purchased from Sigma-Aldrich Co. LLC. The negative control used was MISSION(R) pLKO.1-puro Non-Mammalian shRNA Control Plasmid DNA (SHC002).

For the expression of shRNA, LentiX-293 cells were cotransfected with the plasmid and ViraPower Lentiviral Expression System (Life Technologies Corp.) using Lipofectamine 2000 (Invitrogen Corp.) to prepare lentivirus for transduction with the shRNA expression cassette. The prepared lentivirus was allowed to infect each cell line, and infected cells were selected using puromycin. After selection for 3 days to 1 week, the knockdown of the PTDSS1 gene at the mRNA level was confirmed in the cells of each line.

Significant inhibition of cell growth was observed in the PTDSS2 deletion lines by the RNA interference of PTDSS1. The inhibition of cell growth by the RNA interference of PTDSS1 was not observed in the cell lines having no deletion in PTDSS2 (Figure 4).

### (Inhibition of phosphatidylserine production by PTDSS1 knockdown in PTDSS2 deletion line)

PTDSS2 heterozygous deletion line ZR-75-1 cells or PTDSS2 wild-type HCT116 cells were inoculated onto a 96-well plate. On the next day, the medium was replaced with MEM (10% dialyzed bovine serum) containing 5 µCi/ml of L-[U-¹⁴C]-serine, and the cells were cultured for 30 minutes. The cells were washed twice with PBS. Then, methanol was added thereto, and the mixture was left standing for 30 minutes. Methanol was recovered and mixed with methyl tert-butyl ether, and the mixture was left standing for 5 minutes. Water was further added thereto, and the mixture was mixed and then left standing for 10 minutes. The mixed solution was centrifuged at 3000 rpm for 10 minutes, and the organic layer was extracted. The radioactivity of the organic layer was measured using a liquid scintillation counter. Alternatively, the organic layer was separated by thin layer chromatography (separating solvent: 1-butanol:acetic acid:water = 3:1:1), followed by autoradiography. The number of cells inoculated was set to 7.5 × 10⁴ cells/well for measurement in a liquid scintillation counter and 2 × 10⁴ cells/well for the analysis by thin layer chromatography.

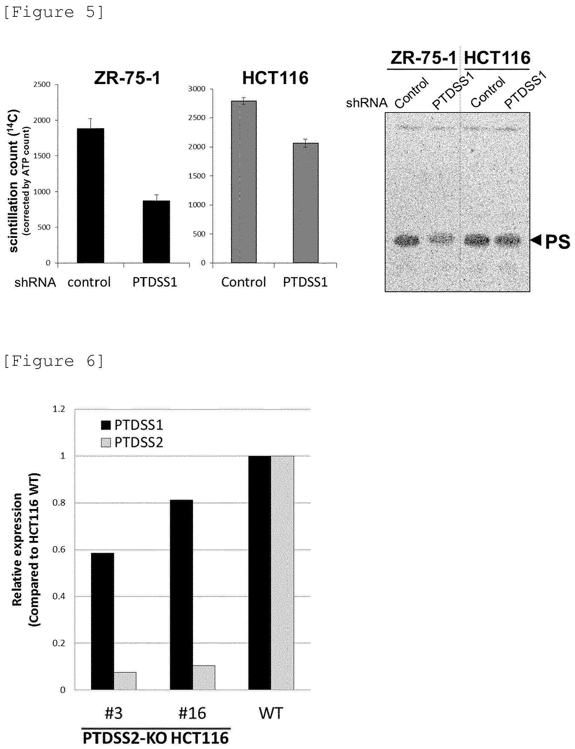

In the PTDSS2 heterozygous deletion line, the uptake of serine into lipid and the production of phosphatidylserine were more strongly inhibited by the RNA interference of PTDSS1, as compared with the PTDSS2 wild-type cell line (Figure 5).

### (Effect of PTDSS1 knockdown on PTDSS2 gene-disrupted (PTDSS2-KO) HCT116 cell)

For the RNA interference of PTDSS1, ON-TARGET plus SMARTpool siRNA was purchased from Dharmacon, Inc. and used. The cells were transfected with PTDSS1 siRNA (catalog No: L-008568-00) or nontargeting siRNA (catalog No: D-001810-10-50) using Lipofectamine RNAiMAX (Invitrogen Corp., 13778-150).

For the observation of serine uptake into lipid, the transfected cells of each line were inoculated at 4 × 10⁴ cells/well onto a 96-well plate, and other procedures were performed in the same way as above.

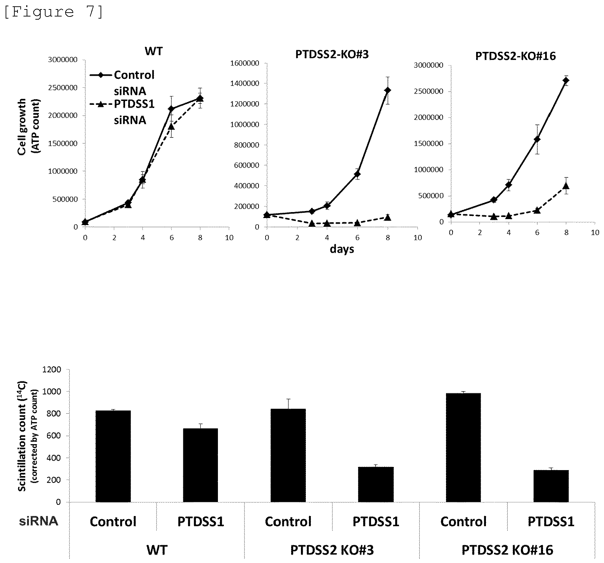

In the PTDSS2-KO HCT116 cells (PTDSS2-KO #3 and PTDSS2-KO #16), significant inhibition of growth was observed by RNA interference of PTDSS1, and the uptake of serine into lipid was also strongly inhibited. By contrast, in the parent line HCT116 cells (WT), the inhibition of growth by RNA interference of PTDSS1 was not observed, and the uptake of serine into lipid was also weakly inhibited (Figure 7).

### Industrial Applicability

The present invention provides a therapeutic strategy for specifically targeting cancer cells having the functional suppression of PSS1. In the present invention, it has been found that PSS1 and PSS2 are in a synthetic lethal relationship, and treatment inhibiting PSS1 serves as a promising approach for the treatment of cancer having PSS2 deletion. It has also been revealed that this therapeutic strategy permits efficient treatment based on companion diagnostics because a PSS1 inhibitor can be administered to a cancer patient selected by using the functional suppression of PSS2 as an index. Thus, the present invention can make a significant contribution to the medical field, particularly, the field of cancer treatment.

### Free Text of Sequence Listing

SEQ ID NO: 1: PSS1 mRNA encoding the PSS1 protein (SEQ ID NO: 2).
SEQ ID NO: 3: PSS2 mRNA encoding the PSS2 protein (SEQ ID NO: 4).

## Claims

1. A method for predicting responsiveness to the treatment of cancer with a phosphatidylserine synthase 1 inhibitor, comprising providing a biological sample derived from a subject, detecting the presence or absence of the functional suppression of phosphatidylserine synthase 2 in the biological sample, and thereby determining that the subject for whom the functional suppression of phosphatidylserine synthase 2 has been detected has responsiveness to the treatment of cancer with a phosphatidylserine synthase 1 inhibitor.

2. A method for selecting a subject for the treatment of cancer with a phosphatidylserine synthase 1 inhibitor, comprising providing a biological sample derived from a subject, detecting the presence or absence of the functional suppression of phosphatidylserine synthase 2 in the biological sample, and selecting the subject for whom the functional suppression of phosphatidylserine synthase 2 has been detected as a subject for the treatment of cancer with a phosphatidylserine synthase 1 inhibitor.

3. A method for treating cancer having the functional suppression of phosphatidylserine synthase 2, comprising providing a biological sample derived from a subject, detecting the presence or absence of the functional suppression of phosphatidylserine synthase 2 in the biological sample, and administering a phosphatidylserine synthase 1 inhibitor to the subject for whom the functional suppression of phosphatidylserine synthase 2 has been detected.

4. A method for treating cancer having the functional suppression of phosphatidylserine synthase 2, comprising administering a phosphatidylserine synthase 1 inhibitor to a subject having the functional suppression of phosphatidylserine synthase 2 in a biological sample derived from the subject.

5. A method for screening for a compound for use in the treatment of cancer having the functional suppression of phosphatidylserine synthase 2, the method comprising the step of selecting the compound by determining whether or not it inhibits phosphatidylserine synthase 1 as an index.

6. The method according to any one of claims 1 to 5, wherein the functional suppression of phosphatidylserine synthase 2 is the homozygous deletion or heterozygous deletion of the phosphatidylserine synthase 2 gene.

7. A therapeutic agent for cancer having the functional suppression of phosphatidylserine synthase 2, comprising a phosphatidylserine synthase 1 inhibitor.

8. The therapeutic agent for cancer according to claim 7, wherein the functional suppression of phosphatidylserine synthase 2 is the homozygous deletion or heterozygous deletion of the phosphatidylserine synthase 2 gene.

9. A method for detecting the presence or absence of the functional suppression of phosphatidylserine synthase 2 in a biological sample derived from a subject in order to select a subject for the treatment of cancer with a phosphatidylserine synthase 1 inhibitor.


SEQUENCE LISTING

<110>  DAIICHI SANKYO COMPANY LIMITED

<120>  A method of predicting a responsiveness to PSS1 inhibitors

<130>  FP1604

<150>  JP2015-052062
<151>  2015-03-16

<160>  4

<170>  PatentIn version 3.5

<210>  1
<211>  2576
<212>  DNA
<213>  Homo sapiens

<220>
<221>  misc_feature
<223>  PSS1 mRNA encoding PSS1 protein (SEQ ID NO.2)


<220>
<221>  CDS
<222>  (156)..(1577)

<400>  1
   tctcgcgcct gtccttccct ctgctcccag cctttgctgg gcgccagacc cggctttgcc      60
   gtccggctat tagcctactg tggctagtca cccccggggt cccggccttc tcgggctggg     120
   gccgccgcca ccgcggcagg acggggaggc gggcc atg gcg tcc tgc gtg ggg        173
   agc cgg acc cta agc aag gat gat gtg aac tac aaa atg cat ttc cgg       221
   atg atc aac gag cag caa gtg gag gac atc acc att gac ttc ttc tac       269
   cgg ccg cat acc atc acc ctg ctc agc ttc acc atc gtc agc ctc atg       317
   tac ttc gcc ttt acc agg gat gac tct gtt cca gaa gac aac atc tgg       365
   aga ggc atc ctc tct gtt att ttc ttc ttt ctt atc atc agt gtg tta       413
   gct ttc ccc aat ggt ccg ttc act cga cct cat cca gcc tta tgg cga       461
   atg gtt ttt gga ctc agt gtg ctc tac ttc ctg ttc ctg gta ttc cta       509
   ctc ttc ctg aat ttc gag cag gtt aaa tct cta atg tat tgg cta gat        557
   cca aat ctt cga tac gcc aca agg gaa gca gat gtc atg gag tat gct        605
   gtg aac tgc cat gtg atc acc tgg gag agg att atc agc cac ttt gat        653
   att ttt gca ttt gga cat ttc tgg ggc tgg gcc atg aag gcc ttg ctg        701
   atc cgt agt tac ggt ctc tgc tgg aca atc agt att acc tgg gag ctg        749
   act gag ctc ttc ttc atg cat ctc ctc ccc aat ttt gcc gag tgc tgg        797
   tgg gat caa gtc att ctg gac atc ctg ttg tgc aat ggc ggt ggc att        845
   tgg ctg ggc atg gtc gtt tgc cgg ttt tta gag atg agg act tac cac        893
   tgg gca agc ttc aag gac att cat acc acc acc ggg aag atc aag aga        941
   gct gtt ctg cag ttc act cct gct agc tgg acc tat gtt cga tgg ttt        989
   gac ccc aaa tct tct ttt cag aga gta gct gga gtg tac ctt ttc atg        1037
   atc atc tgg cag ctg act gag ttg aat acc ttc ttc ttg aag cat atc        1085
   ttt gtg ttc caa gcc agt cat cca tta agt tgg ggt aga att ctc ttt        1133
   att ggt ggc atc aca gct ccc aca gtg aga cag tac tac gct tac ctc        1181
   acc gac aca cag tgc aag cgc gta gga aca caa tgc tgg gtg ttt ggg        1229
   gtc att ggt ttc ctg gag gcc att gtt tgc ata aaa ttt gga caa gat     1277
   ctc ttc tct aag acc caa ata ctc tat gtt gtg ctt tgg ctt ctt tgc     1325
   gtg gct ttc acc act ttc ctc tgt ctg tac ggc atg att tgg tat gca     1373
   gaa cac tat ggt cac cga gaa aag acc tac tcg gag tgt gaa gat ggc     1421
   acc tac agt cca gag atc tcc tgg cat cac agg aaa ggg aca aaa ggt     1469
   tct gaa gac agc cca ccc aag cat gca ggc aac aac gaa agc cat tct     1517
   tcc agg aga agg aat cgg cat tcc aag tca aaa gtc acc aat ggc gtt     1565
   gga aag aaa tga aaaaccctgg ttaatcaaag atgttccaga gtgcctagaa     1617
   ctgagaggga aatggaactc atttggaact ccccgtgagg aggtcgaggc gcacagggca     1677
   agcaggaaga ggcgagggca cttgggggtc attatttgag atcgtaagtc ttgtttccca     1737
   cagacctggc cgcgtcaggc agatcatcgc ctggggggcc tttgccaacg tggggtctct     1797
   tctaacttca gcacttgaca tgcggtcacc ggtggcagcg cggtgtgttg aagggaaacg     1857
   gtagctattc attcacagtt gccaagagca gctccgcgcc tgctggatcg tggatgcagc     1917
   gtaaacatct tccttcagac gaggcattaa ccccatggtt aatggactgg tcaccagttt     1977
   ttattttatt tttatgaatc tacctttcca ttgattgatt taagttcagg ccacttttct     2037
   gtcttttatt tggttactgt tgttatttgt ttttaagtta ggatgctttt taacagcctt     2097
   tagaagccgc tgctgaaatt gatactgggg gaagggttcc ccttccttct agagcagaaa     2157
   agggagagag gtgttgtatt cctgtttggt aacctcagtc tcctgtaaga cctcctacca     2217
   catggcgagt atacaccaat caggagaggg tagctgcctg cataggagcc tcgcttccga     2277
   ttattccctt cccaatatta ttcatccaga cttagccaca gtgcacaaaa gcaaacctgc     2337
   tagagaggca gtgaacacca cagcttctcc ccagctaggt gccttttaca tcgggtttgt     2397
   tctccttcca tggtgtgttg ctgacattgt cactgagtcc catgtgaggt gctggtgagt     2457
   attacctttc atctgtgcca tgctctagaa ccttgacctt gatagttcac cacctctgat     2517
   ggatccctgt tttaaataaa aacgattcac tttaaagcct atcaaaaaaa aaaaaaaaa     2576

<210>  2
<211>  473
<212>  PRT
<213>  Homo sapiens

<400>  2

<210>  3
<211>  2473
<212>  DNA
<213>  Homo sapiens

<220>
<221>  misc_feature
<223>  PSS2 mRNA encoding PSS2 protein (SEQ ID NO.4)

<220>
<221>  CDS
<222>  (177)..(1640)

<400>  3
caatgcaccg cacacccttt actggccggc cccgcgctgc tctcctaaga ccccgcgggc      60

cagcgccgcg accccttccc agcgctcctc gcgctgtgtg cggcgcgtcc tctcgccggt     120

gacccggtgt gcgtggggtc gaggcgccgg gcggagtggc tccgggccga aacgcc atg      179
                                                              Met
                                                              1

cgg agg ggc gag cgc agg gac gcc gga ggt ccg cgg ccc gag tcc ccg        227
Arg Arg Gly Glu Arg Arg Asp Ala Gly Gly Pro Arg Pro Glu Ser Pro
            5                   10                  15

gtg ccc gcg ggc agg gcc tcg ctg gag gag ccg cct gac ggg ccg tct        275
Val Pro Ala Gly Arg Ala Ser Leu Glu Glu Pro Pro Asp Gly Pro Ser
        20                  25                  30

gcc ggc caa gcc acc ggg ccg ggc gag ggc cgc cgc agc acc gag tcc        323
Ala Gly Gln Ala Thr Gly Pro Gly Glu Gly Arg Arg Ser Thr Glu Ser
    35                  40                  45

gag gtc tac gac gac ggc acc aac acc ttc ttc tgg cga gcc cac acc        371
Glu Val Tyr Asp Asp Gly Thr Asn Thr Phe Phe Trp Arg Ala His Thr
50                  55                  60                  65

tta acc gtg ctc ttc atc ctc acc tgt acg ctt ggc tat gtg acg ctg        419
Leu Thr Val Leu Phe Ile Leu Thr Cys Thr Leu Gly Tyr Val Thr Leu
                70                  75                  80

ctg gag gaa aca cct cag gac acg gcc tac aac acc aag aga ggt att        467
Leu Glu Glu Thr Pro Gln Asp Thr Ala Tyr Asn Thr Lys Arg Gly Ile
            85                  90                  95

gtg gcc agt att ttg gtt ttc tta tgt ttt gga gtc aca caa gct aaa        515
Val Ala Ser Ile Leu Val Phe Leu Cys Phe Gly Val Thr Gln Ala Lys
        100                 105                 110

gac ggg cca ttt tcc aga cct cat cca gct tac tgg agg ttt tgg ctc        563
Asp Gly Pro Phe Ser Arg Pro His Pro Ala Tyr Trp Arg Phe Trp Leu
    115                 120                 125

tgc gtg agt gtg gtc tac gag ctg ttt ctc atc ttt ata ctc ttc cag        611
Cys Val Ser Val Val Tyr Glu Leu Phe Leu Ile Phe Ile Leu Phe Gln
130                 135                 140                 145

act gtc cag gac ggc cgg cag ttt cta aag tat gtt gac ccc aag ctg        659

Thr Val Gln Asp Gly Arg Gln Phe Leu Lys Tyr Val Asp Pro Lys Leu
                150                 155                 160

gga gtc cca ctg cca gag aga gac tac ggg gga aac tgc ctc atc tac      707
Gly Val Pro Leu Pro Glu Arg Asp Tyr Gly Gly Asn Cys Leu Ile Tyr
            165                 170                 175

gac cca gac aat gag act gac ccc ttt cac aac atc tgg gac aag ttg      755
Asp Pro Asp Asn Glu Thr Asp Pro Phe His Asn Ile Trp Asp Lys Leu
        180                 185                 190

gat ggc ttt gtt ccc gcg cac ttt ctt ggc tgg tac ctg aag acc ctg      803
Asp Gly Phe Val Pro Ala His Phe Leu Gly Trp Tyr Leu Lys Thr Leu
    195                 200                 205

atg atc cga gac tgg tgg atg tgc atg atc atc agc gtg atg ttc gag      851
Met Ile Arg Asp Trp Trp Met Cys Met Ile Ile Ser Val Met Phe Glu
210                 215                 220                 225

ttc ctg gag tac agc ctg gag cac cag ctg ccc aac ttc agc gag tgc      899
Phe Leu Glu Tyr Ser Leu Glu His Gln Leu Pro Asn Phe Ser Glu Cys
                230                 235                 240

tgg tgg gat cac tgg atc atg gac gtg ctc gtc tgc aac ggg ctg ggc      947
Trp Trp Asp His Trp Ile Met Asp Val Leu Val Cys Asn Gly Leu Gly
            245                 250                 255

atc tac tgc ggc atg aag acc ctt gag tgg ctg tcc ctg aag acg tac      995
Ile Tyr Cys Gly Met Lys Thr Leu Glu Trp Leu Ser Leu Lys Thr Tyr
        260                 265                 270

aag tgg cag ggc ctc tgg aac att ccg acc tac aag ggc aag atg aag      1043
Lys Trp Gln Gly Leu Trp Asn Ile Pro Thr Tyr Lys Gly Lys Met Lys
    275                 280                 285

agg atc gcc ttc cag ttc acg ccg tac agc tgg gtt cgc ttc gag tgg      1091
Arg Ile Ala Phe Gln Phe Thr Pro Tyr Ser Trp Val Arg Phe Glu Trp
290                 295                 300                 305

aag ccg gcc tcc agc ctg cgt cgc tgg ctg gcc gtg tgc ggc atc atc      1139
Lys Pro Ala Ser Ser Leu Arg Arg Trp Leu Ala Val Cys Gly Ile Ile
                310                 315                 320

ctg gtg ttc ctg ttg gca gaa ctg aac acg ttc tac ctg aag ttt gtg      1187
Leu Val Phe Leu Leu Ala Glu Leu Asn Thr Phe Tyr Leu Lys Phe Val
            325                 330                 335

ctg tgg atg ccc ccg gag cac tac ctg gtc ctc ctg cgg ctc gtc ttc      1235
Leu Trp Met Pro Pro Glu His Tyr Leu Val Leu Leu Arg Leu Val Phe
        340                 345                 350

ttc gtg aac gtg ggt ggc gtg gcc atg cgt gag atc tac gac ttc atg      1283
Phe Val Asn Val Gly Gly Val Ala Met Arg Glu Ile Tyr Asp Phe Met
    355                 360                 365

gat gac ccg aag ccc cac aag aag ctg ggc ccg cag gcc tgg ctg gtg      1331
Asp Asp Pro Lys Pro His Lys Lys Leu Gly Pro Gln Ala Trp Leu Val
370                 375                 380                 385

gcg gcc atc acg gcc acg gag ctg ctc atc gtg gtg aag tac gac ccc      1379
Ala Ala Ile Thr Ala Thr Glu Leu Leu Ile Val Val Lys Tyr Asp Pro
                390                 395                 400

cac acg ctc acc ctg tcc ctg ccc ttc tac atc tcc cag tgc tgg acc     1427
His Thr Leu Thr Leu Ser Leu Pro Phe Tyr Ile Ser Gln Cys Trp Thr
            405                 410                 415

ctc ggc tcc gtc ctg gcg ctc acc tgg acc gtc tgg cgc ttc ttc ctg     1475
Leu Gly Ser Val Leu Ala Leu Thr Trp Thr Val Trp Arg Phe Phe Leu
        420                 425                 430

cgg gac atc aca ttg agg tac aag gag acc cgg tgg cag aag tgg cag     1523
Arg Asp Ile Thr Leu Arg Tyr Lys Glu Thr Arg Trp Gln Lys Trp Gln
    435                 440                 445

aac aag gat gac cag ggc agc acc gtc ggc aac ggg gac cag cac cca     1571
Asn Lys Asp Asp Gln Gly Ser Thr Val Gly Asn Gly Asp Gln His Pro
450                 455                 460                 465

ctg ggg ctg gac gaa gac ctg ctg ggg cct ggg gtg gcc gag ggc gag     1619
Leu Gly Leu Asp Glu Asp Leu Leu Gly Pro Gly Val Ala Glu Gly Glu
                470                 475                 480

gga gca cca act cca aac tga cctgggccgt ggctgcctcg tgagcctccc     1670
Gly Ala Pro Thr Pro Asn
            485

agagcccagg cctccgtggc ctcctcctgt gtgagtccca ccaggagcca cgtgcccggc     1730

cttgccctca aggttttttg cttttctcct gtgcacctgg cgaggctgaa ggcgaggggt     1790

ggaggaggcc ccagcacagc ctcatctcca tgtgtacacg tgtgtacgtg tgtatgcgtg     1850

tgtgtacgcg tgtgtacgcg cgtgtgtaca catgcgtggc cgcctgtggt gtgcacgtgt     1910

gctctgggct ccgaggcttc tccagagctg ggagctggct ggcgtggcaa gggcatgctc     1970

tggggcagtg tgtccctcag gaaccagggt cctccctccc ctttctgcct ggtcagcccc     2030

gtggcctctg gcccaccaag ctccctgtca cccagccatg gtgtggtcca ggcagggaca     2090

tctcggtacc ctttctgcac tccgtgggcc ctgggtgcgc tgaggcctgg aggcgtctac     2150

actggctcca catccacttc ccccgcagct cgtgtgggcg ctcgtccaca aacactccgt     2210

ggctgagagg cagcggatcc aggcagcgat gctgagccac ctcctccgag ccttcctttc     2270

acacagacca ccccggagga cacgtggatg atggggtcag agatcactga gctgcccctc     2330

aagggggcct ggaacccggg tgctggggtc atgctgcctc cgtggctcca aggtgagggt     2390

catcttcacg agcaaagaga accaataaag tgacaacgaa cgtctgaggc ttccaaaaaa     2450

aaaaaaaaaa aaaaaaaaaa aaa                                           2473

<210>  4
<211>  487
<212>  PRT
<213>  Homo sapiens

<400>  4

Met Arg Arg Gly Glu Arg Arg Asp Ala Gly Gly Pro Arg Pro Glu Ser
1               5                   10                  15

Pro Val Pro Ala Gly Arg Ala Ser Leu Glu Glu Pro Pro Asp Gly Pro
            20                  25                  30

Ser Ala Gly Gln Ala Thr Gly Pro Gly Glu Gly Arg Arg Ser Thr Glu
        35                  40                  45

Ser Glu Val Tyr Asp Asp Gly Thr Asn Thr Phe Phe Trp Arg Ala His
    50                  55                  60

Thr Leu Thr Val Leu Phe Ile Leu Thr Cys Thr Leu Gly Tyr Val Thr
65                  70                  75                  80

Leu Leu Glu Glu Thr Pro Gln Asp Thr Ala Tyr Asn Thr Lys Arg Gly
                85                  90                  95

Ile Val Ala Ser Ile Leu Val Phe Leu Cys Phe Gly Val Thr Gln Ala
            100                 105                 110

Lys Asp Gly Pro Phe Ser Arg Pro His Pro Ala Tyr Trp Arg Phe Trp
        115                 120                 125

Leu Cys Val Ser Val Val Tyr Glu Leu Phe Leu Ile Phe Ile Leu Phe
    130                 135                 140

Gln Thr Val Gln Asp Gly Arg Gln Phe Leu Lys Tyr Val Asp Pro Lys
145                 150                 155                 160

Leu Gly Val Pro Leu Pro Glu Arg Asp Tyr Gly Gly Asn Cys Leu Ile
                165                 170                 175

Tyr Asp Pro Asp Asn Glu Thr Asp Pro Phe His Asn Ile Trp Asp Lys
            180                 185                 190

Leu Asp Gly Phe Val Pro Ala His Phe Leu Gly Trp Tyr Leu Lys Thr
        195                 200                 205

Leu Met Ile Arg Asp Trp Trp Met Cys Met Ile Ile Ser Val Met Phe
    210                 215                 220

Glu Phe Leu Glu Tyr Ser Leu Glu His Gln Leu Pro Asn Phe Ser Glu
225                 230                 235                 240

Cys Trp Trp Asp His Trp Ile Met Asp Val Leu Val Cys Asn Gly Leu
                245                 250                 255

Gly Ile Tyr Cys Gly Met Lys Thr Leu Glu Trp Leu Ser Leu Lys Thr
            260                 265                 270

Tyr Lys Trp Gln Gly Leu Trp Asn Ile Pro Thr Tyr Lys Gly Lys Met
        275                 280                 285

Lys Arg Ile Ala Phe Gln Phe Thr Pro Tyr Ser Trp Val Arg Phe Glu
    290                 295                 300

Trp Lys Pro Ala Ser Ser Leu Arg Arg Trp Leu Ala Val Cys Gly Ile
305                 310                 315                 320

Ile Leu Val Phe Leu Leu Ala Glu Leu Asn Thr Phe Tyr Leu Lys Phe
                325                 330                 335

Val Leu Trp Met Pro Pro Glu His Tyr Leu Val Leu Leu Arg Leu Val
            340                 345                 350

Phe Phe Val Asn Val Gly Gly Val Ala Met Arg Glu Ile Tyr Asp Phe
        355                 360                 365

Met Asp Asp Pro Lys Pro His Lys Lys Leu Gly Pro Gln Ala Trp Leu
    370                 375                 380

Val Ala Ala Ile Thr Ala Thr Glu Leu Leu Ile Val Val Lys Tyr Asp
385                 390                 395                 400

Pro His Thr Leu Thr Leu Ser Leu Pro Phe Tyr Ile Ser Gln Cys Trp
                405                 410                 415

Thr Leu Gly Ser Val Leu Ala Leu Thr Trp Thr Val Trp Arg Phe Phe
            420                 425                 430

Leu Arg Asp Ile Thr Leu Arg Tyr Lys Glu Thr Arg Trp Gln Lys Trp
        435                 440                 445

Gln Asn Lys Asp Asp Gln Gly Ser Thr Val Gly Asn Gly Asp Gln His
    450                 455                 460

Pro Leu Gly Leu Asp Glu Asp Leu Leu Gly Pro Gly Val Ala Glu Gly
465                 470                 475                 480

Glu Gly Ala Pro Thr Pro Asn
                485